# EUROPEAN PATENT SPECIFICATION

(11) **EP 1 501 405 B1**
(45) Date of publication and mention of the grant of the patent: **28.07.2010**
(21) Application number: 03717172.5
(22) Date of filing: 29.04.2003
(51) Int. Cl.: A61B 5/00

(54) **NEEDLE INSERTION SENSOR**
NADELEINFÜHRUNGSSENSOR
SONDE D'INTRODUCTION D'AIGUILLE

(30) Priority: 30.04.2002 DK 200200650
(43) Date of publication of application: 02.02.2005
(73) Proprietor: NOVO NORDISK A/S, 2880 Bagsværd (DK)
(72) Inventor: CHRISTENSEN, Lars, Hofmann, DK-4040 Jyllinge (DK); POULSEN, Jens, Ulrik, DK-2830 Virum (DK); SIMONSEN, Jan, H., DK-7600 Struer (DK)
(86) International application number: PCT/DK2003/000275
(87) International publication number: WO 2003/092487

(56) References cited:
- GB-A- 2 309 644
- US-A- 4 871 351
- US-A- 5 741 211
- US-A- 5 971 963
- US-A1- 2002 002 326

## Description

### Field of the invention

The present invention relates to a doser comprising a syringe having a needle which extends outside the doser, which comprises an engagement face in the vicinity of the needle so that the engagement face rests against the tissue into which the injection is inserted.

### Background of the invention

Injection devices for multiple use having an exchangeable insulin ampoule have been developed, which calculate an optimum dose of medicine for a given patient (WO 00/32088). This calculation can take the patient's health, food habits and recordings of previously administered doses into consideration. For this determination of dose it is necessary to know the patient's received dose precisely to avoid overdosing or underdosing, as this may have fatal consequences for the diabetic.

GB-A-2 309 644 discloses a device, which is particularly for insulin measurement/injection. The device comprises a housing having means for producing and maintaining a skin fold, a cannula and sensor/probe for metabolic measurement, means for moving the cannula etc. towards and away from the skin fold, a measuring device consisting of a light source, light receiver and control unit, and an injection/metering device. The sensor may include a light conducting fibre leading to a colour changing reaction layer.

### Object of the invention

The object of the invention is to utilize the known sophisticated electronics fo even better information of the user on the basis of the provision of additional input signals to the electronics, without the user having to do anything else than he/she normally does.

This object is obtained in that measurements of the heart rate are accomplished in that signal processing known per se is used in combination with detector (means for this measurement being provided on said engagement face.

Thereby signals are obtained that are related to heart activity and by expanding the doser with additional electrode means provided on the handle, it is possible to obtain electrocardiograms. Preferably additional electrode means are provided that can be located elsewhere on the body and, either via wires or wireless communication links, they are connected to the doser.

Preferably electrical signals are used, but it is also an option to use optical signals, like eg in connection with BGM measurements (Blood Glucose Measurement).

Typically, the impedance is measured in the tissue touched by the engagement face of the doser or, alternatively, light is used. The signals may be modulated to avoid noise from the surroundings.

The doser of the invention is based on the finding that merely by a very simple detection of heart signals, it is possible to considerably improve the applicability. This is due to the fact that the prior art calculating circuits are very sophisticated, ia with self-learning software routines that can either be executed within the doser as such or be executed in a large, external computer connected to the doser via a wire or a wireless communication link. In this manner the doser may have very large signal-processing capacity and therefore the doser can advantageously be provided with means for receiving external signals.

By the provision of extra electrode means at the end opposite that end of the doser which is in contact with the skin of the stomach of the user, it is accomplished that the patient's hand touches the additional electrode means, whereby the measurement will take place through the patient's heart region. It is therefore possible, by simple means, to considerably broaden the applicability. By supplementing with light detection it is also possible to perform a blood glucose measurement (BGM) in connection with the doser that thus also lends itself for use as hypoglycemia alarm.

A further advantageous use of the doser of the invention relates to administering of the dosis which is injected. It is assumed in the calculations that all the medicine discharged from the ampoule is administered to the patient. When replacing the reservoir it is essential to drain the injection doser needle as well as the syringe for any possible air, as this might fill the patient's veins. Consequently, the operator usually performs a first time shot e.g. into a sponge after reservoir replacement to make sure that there is no air left in the needle or eventual in the syringe. This shot will inappropriately be recorded in the doser as an injection shot into the patient, and it will therefore be necessary to observe whether the needle is inserted into biological tissue, e.g. a human body. This drawback is avoided by the doser according to the invention that can very reliably detect whether dosis is administered to biological tissue, since the decision can be taken on the basis of the detection of heart rate.

### Brief description of the drawings

The accompanying drawings illustrate the present invention by way of the embodiments in which:
Fig. 1 shows an embodiment of the doser according to the present invention where the engagement face rests against skin.
Fig. 2 illustrates an embodiment of the invention in detail and with sensor means on the injection button.
Fig. 3 shows an embodiment of a sensor with two electrodes according to the present invention.
Fig. 4 illustrates a QRS-pass as seen on a typical electrocardiogram.

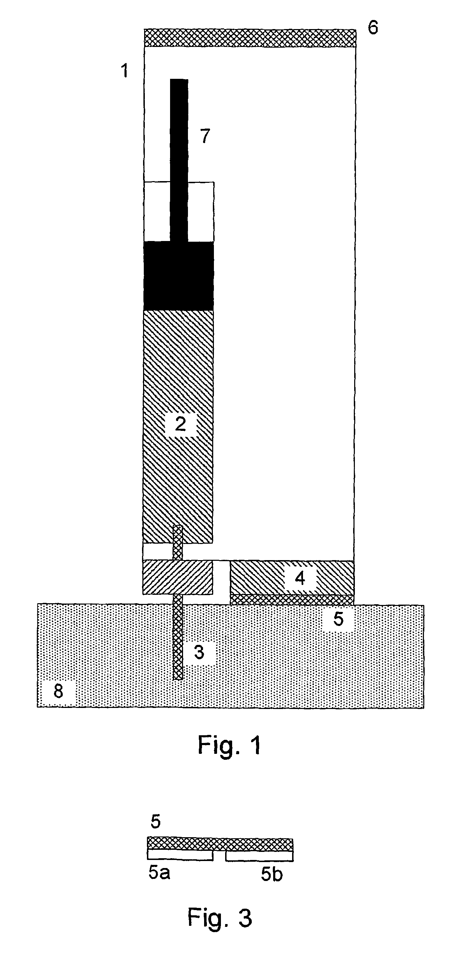

Figure 1 shows detector means (5) on the engagement face (4) of a doser that naturally contacts the skin (8) in normal use.

The doser (1) comprises a syringe (2) having a needle (3) that generally extends outside the doser that comprises an engagement face (4) in the vicinity of the needle, so that the engagement face rests against the surface of the tissue (8) into which the needle has been inserted. The engagement face has one or more closely spaced electrodes connected to detector means for measuring electrical pulses.

Figure 3 shows an example of such a sensor (5) with two closely spaced electrodes (5a, 5b).

In an embodiment, the electrical impedance between such two electrodes is measured, and it can hereby be determined whether the doser is in engagement with human tissue. The doser may thus be adapted for specifically recording the amount of administered dose to a tissue type having human characteristics.

According to a preferred feature of the invention, the heart rate of the patient can be determined by a continuous recording of the impedance of the skin, which gives a further indication of live tissue not obtained in prior art whereby a more safe decision can be made to determine whether the insulin discharge takes place in a patient.

More importantly, the detector means according to the invention can be used to determine the heart rate itself, even if a person skilled in the art would expect that heart rate signals obtained in a way according to the invention - at least to a certain extent - would be of too low a quality to make a decision on something as important as the heart rate.

By combining the detector means according to the invention with modern signal shaping routines, e.g. comprising naural network analysis (see WO 02/069798) reliable results can be obtained on the basis of less reliable detector signals whereby the doser of the invention is operable even under difficult conditions.

If the patient's skin is wet, the impedance between the electrodes of the engagement face will be measured so low that it is determined by the doser that human-like skin is not involved. In this case it is therefore expedient that the needle can comprise a sensor, e.g. an electrode, thereby allowing a measurement between the sensor of the engagement face (5) and the sensor of the needle. This provides an extra possibility of reliable measurement results.

In a preferred embodiment a sensor is embedded also in the handle of the doser (6). This allows for measurement between it and the sensor of the engagement face (5) and/or the sensor of the needle (3). According to a preferred embodiment of the invention, this makes the heart rate signals useable for obtaining electrocardiogram signals, as will be described below.

Since an injection is typically made in the patient's thigh or pit of the stomach, the current path between the sensors of the doser at the end of the engagement face and the handle of the doser will run through the patient's heart region and one arm, which enables mapping of the heart rate as well as diastole and systole of the patient's ventricles. This possibility allows diagnosis of the patient's circulatory state, that is, if the patient himself operates the doser.

In this embodiment, the doser may moreover be adapted for calculating correlation between a sensor signal originating from the engagement face (5) and the sensor signal of the handle (6) so as to determine whether these pulse rates are consistent. If these two signals do not resemble each other, it will mean that the patient does not operate the apparatus himself, but that the apparatus is operated by another person, e.g. a nurse. The doser can thus determine on the basis of this correlation that it is not possible to create a valid pseudo electrocardiogram (ECG).

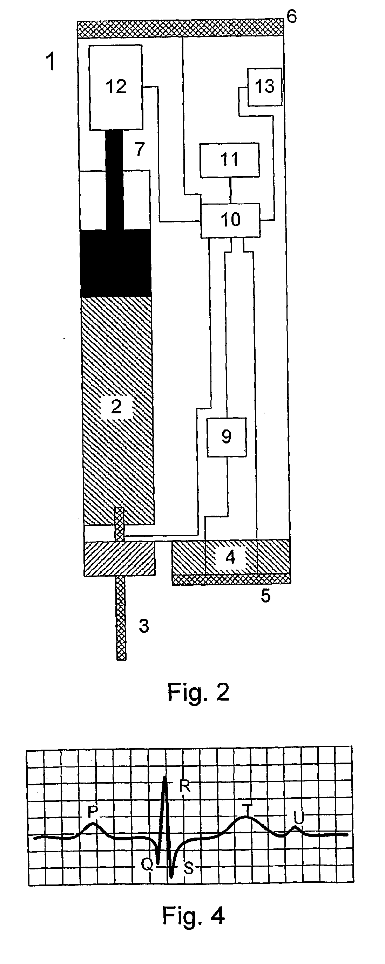

The basics of the doser according to the present invention are shown in figure 2. Signal processing/calculation means (10) is connected with memory storage (11) containing e.g. user information, operating system, executable program, etc.

The processing means may include a microprocessor, an application-specific integrated circuit, or another integrated circuit, a smart card, a general purpose computer adapted by suitable software, or the like. The processing means may be designed to acquire information from the internal sensors/electrodes (3, 5 and 6) as well as from an external communication link (13). The communication link (13) may be any transmission line which may comprise wire and wire-less communication links.

Additionally, the processing means may comprise means to control the generation of control signals to a pump (12), e.g. a DC-driven motor, etc., which may enable an injection by way of moving a piston rod (7) in the syringe (2). Further, a signal device (9) may be present to generate measurement impulses, e.g. a light emitter generator etc.

Creation of a standard ECG signal requires measurement on three points on the patient's body. Even though the number of acceptable sensor points in this embodiment will be three (handle, needle, and engagement face), this method will not always be sufficient to obtain a complete electrocardiogram. Since the needle and engagement face sensor points are spaced closely together, the measurement signal processing requires an unacceptable high signal/noise ratio. To overcome this, it is therefore desirable to ensure that at least one external sensor for this purpose can be connected to the doser. In this embodiment, it will thus be possible to generate a true balanced measuring signal. This connection may be carried out by a physical wire and/or radio communication. To screen noise from the surroundings, it is necessary strongly to filter the resulting signal of especially the 50/60 HZ power supply frequency. The signal may then be chased for a possible useful ECG signal where a QRS course is desired.

In this case, the doser is adapted to recognize the shape of the QRS course of a human heart. To achieve a better noise/signal ratio, the measuring signal may be modulated in frequency to a range, which is discordant relative to the frequency of the power supply, that is, at a frequency which is not a whole numbered multiple of the power supply frequency.

If freedom of movement is desired, this sensor may consist of a wireless electrode having a plurality of electrodes (US 6 073 046) adhered to the patient's chest. By implementation of ECG monitoring by means of the doser, this can completely replace a commonly used ECG apparatus.

It is also to be understood that the communication link 13 can be used to transmit information about the heart rate signals measured to a separate computer. Especially when a neural network is used to enhance the quality of the signal as disclosed in WO 02/069798, it may be expedient to use an external computer with large processing power instead of building in an internal computer having limited processing power.

It is moreover possible to replace the electrodes by an emitter/detector or receiver/transducer so as to enable emission and reception of optical signals and ultrasonic signals, respectively.

The use of optical signals provides a new possibility for the field of use of the doser for measuring physiological parameters. For an effective treatment of diabetes, it is necessary to know the patient's content of glucose in the blood (called BGM below), since this quantity influences the determination of the insulin dose amount. Currently, much research is focused on intravenous measuring methods which can determine the BGM by means of optics (US 6 043 492) or by means of electrochemical sensors (US 5 954 685). These sensors measure on the patient's skin and therefore eliminate the need for invasion.

The use of such sensors in the doser allows these methods to be employed for carrying out glucometry during injection, without the patient noticing this or performing any other actions. When commencing an injection, the doser may record the BGM with a view to determining the insulin amount and/or recommending an optimum diet for the patient, as described in WO 00/32088.

Some times, however, the patient wants to check the glucose state before he/she decides on insulin injection. If the patient exclusively wants to know the BGM and therefore does not want to insert the needle into the body, it will be desirable that the doser is adapted to accommodate the entire needle so that the needle may be pushed/pulled into the doser, whereby the needle will be hidden at times when it is not needed. As long as the needle does not extend outside the doser, a measurement may be performed simply by keeping the doser with the engagement face against the skin. This simple measuring method will motivate the patient to check the BGM to a greater extent, which can contribute to a better controlled treatment and therefore fewer sufferings because of diabetes over a span of years.

In summary, when detector means are provided on a doser for detecting of heart rate, a lot of new features can be obtained. Obtaining partly or full-scale EKG signals and the option of BGM measurements have been described. It will be understood that these features could also be made use of for making a hand-held hypo-alarm.

## Claims

1. A doser (1) comprising a syringe (2) with a needle (3) which extends beyond the doser, said doser comprising, in the vicinity of the needle, an engagement face (4) for engaging a surface of a tissue (8) to which the needle is to be inserted and detector means, **characterized in that** detector means (5) capable of detecting heart rate are provided on said engagement face.

2. A doser according to claim 1, **characterized in that** the doser comprises additional electrode means capable of detecting heart rate.

3. A doser according to claim 2, **characterized in that** the additional electrode means are provided on a handle (6) of the doser.

4. A doser according to claim 1-3, **characterized in that** the needle comprises additional electrode means.

5. A doser according to claims 1-4, **characterized in that** the electrode means are sensitive to electrical signals.

6. A doser according to claims 1-4, **characterized in that** the detector means are sensitive to optical signals.

7. A doser according to claim 5, **characterized in that** the doser comprises electrical circuits for generating and for receiving electrical signals for measuring the impedance between electrodes of said electrode means.

8. A doser according to claim 6, **characterized in that** the doser comprises an emitter for generating and emitting light and detector means for detecting light.

9. A doser according to claims 7-8, **characterized in that** the signals are modulated.

10. A doser according to claims 7-8, **characterized in that** the doser contains a calculating circuit and a storage circuit adapted to process and store the value of said signals.

11. A doser according to claims 1-10, **characterized in that** the doser has means for receiving external signals, and that the circuit in the doser is adapted to combine the external signals with signals from said detector means.

12. A doser according to claims 1-11, **characterized in that** the doser contains means for wireless communication.

13. A doser according to claims 1-12, **characterized in that** the detector means is combined with a neural network program means for signal processing.

14. A doser according to claim 13, **characterized in that** the neural network program is executed on an external computer connected to the doser via a wireless communication link.

15. A doser according to 14, **characterized in that** said electrode means comprise a pair of mutually closely spaced electrodes.

16. A doser according to claims 9-13, **characterized in that** the doser is adapted to estimate electrocardiogram signals.

17. A doser according to claims 9-13, **characterized in that** the circuit is adapted to recognize specific signal shapes.

18. A doser according to claims 9-13, **characterized in that** the circuit is adapted to determine pulse rates.

19. A doser according to claim 5, **characterized in that** the circuit is adapted to calculate the blood glucose level.

20. A doser according to claim 19, **characterized in that** the circuit is adapted to calculate doses in dependence on the calculated blood glucose level.

21. A doser according to claim 20, **characterized in that** the circuit is adapted to control the generation of control signals to a pump in the doser.

## Patentansprüche

1. Dosiervorrichtung (1), umfassend eine Spritze (2) mit einer Nadel (3), die sich über die Dosiervorrichtung hinaus erstreckt, wobei die Dosiervorrichtung in der Nähe der Nadel eine Aufnahmefläche (4) zum Aufnehmen einer Oberfläche eines Gewebes (8), in welches die Nadel einzuführen ist, und Erfassungsmittel umfasst, **dadurch gekennzeichnet, dass** Erfassungsmittel (5), die in der Lage sind, die Herzfrequenz zu erfassen, auf der Aufnahmefläche bereitgestellt sind.

2. Dosiervorrichtung nach Anspruch 1, **dadurch gekennzeichnet, dass** die Dosiervorrichtung zusätzliche Elektrodenmittel umfasst, die in der Lage sind, die Herzfrequenz zu erfassen.

3. Dosiervorrichtung nach Anspruch 2, **dadurch gekennzeichnet, dass** die zusätzlichen Elektrodenmittel auf einem Griff (6) der Dosiervorrichtung bereitgestellt sind.

4. Dosiervorrichtung nach Anspruch 1-3, **dadurch gekennzeichnet, dass** die Nadel zusätzliche Elektrodenmittel umfasst.

5. Dosiervorrichtung nach den Ansprüchen 1-4, **dadurch gekennzeichnet, dass** die Elektrodenmittel für elektrische Signale empfänglich sind.

6. Dosiervorrichtung nach den Ansprüchen 1-4, **dadurch gekennzeichnet, dass** die Erfassungsmittel für optische Signale empfänglich sind.

7. Dosiervorrichtung nach Anspruch 5, **dadurch gekennzeichnet, dass** die Dosiervorrichtung elektrische Schaltungen zum Erzeugen und zum Empfangen von elektrischen Signalen zum Messen der Impedanz zwischen Elektroden der Elektrodenmittel umfasst.

8. Dosiervorrichtung nach Anspruch 6, **dadurch gekennzeichnet, dass** die Dosiervorrichtung einen Emitter zum Erzeugen und Emittieren von Licht und Erfassungsmittel zum Erfassen von Licht umfasst.

9. Dosiervorrichtung nach den Ansprüchen 7-8, **dadurch gekennzeichnet, dass** die Signale moduliert werden.

10. Dosiervorrichtung nach den Ansprüchen 7-8, **dadurch gekennzeichnet, dass** die Dosiervorrichtung einen Rechnerschaltkreis und einen Speicherschaltkreis umfasst, die zum Verarbeiten und Speichern des Werts der Signale angepasst sind.

11. Dosiervorrichtung nach den Ansprüchen 1-10, **dadurch gekennzeichnet, dass** die Dosiervorrichtung Mittel zum Empfangen von externen Signalen aufweist, und dass die Schaltung in der Dosiervorrichtung zum Kombinieren der externen Signale mit Signalen von den Erfassungsmitteln angepasst ist.

12. Dosiervorrichtung nach den Ansprüchen 1-11, **dadurch gekennzeichnet, dass** die Dosiervorrichtung Mittel zur drahtlosen Kommunikation enthält.

13. Dosiervorrichtung nach den Ansprüchen 1-12, **dadurch gekennzeichnet, dass** das Erfassungsmittel mit einem Programmmittel für neuronale Netzwerke zur Signalverarbeitung kombiniert ist.

14. Dosiervorrichtung nach Anspruch 13, **dadurch gekennzeichnet, dass** das Programm für neuronale Netzwerke auf einem externen Computer ausgeführt wird, der über einen drahtlosen Kommunikationslink mit der Dosiervorrichtung verbunden ist.

15. Dosiervorrichtung nach Anspruch 14, **dadurch gekennzeichnet, dass** die Elektrodenmittel ein Paar gegenseitig eng beabstandete Elektroden umfassen.

16. Dosiervorrichtung nach den Ansprüchen 9-13, **dadurch gekennzeichnet, dass** die Dosiervorrichtung zum Bewerten von Elektrokardiogrammsignalen angepasst ist.

17. Dosiervorrichtung nach den Ansprüchen 9-13, **dadurch gekennzeichnet, dass** die Schaltung zum Erkennen von spezifischen Signalformen angepasst ist.

18. Dosiervorrichtung nach den Ansprüchen 9-13, **dadurch gekennzeichnet, dass** die Schaltung zum Bestimmen von Pulsfrequenzen angepasst ist.

19. Dosiervorrichtung nach Anspruch 5, **dadurch gekennzeichnet, dass** die Schaltung zum Berechnen des Blutglucosespiegels angepasst ist.

20. Dosiervorrichtung nach Anspruch 19, **dadurch gekennzeichnet, dass** die Schaltung zum Berechnen von Dosen in Abhängigkeit von dem berechneten Blutglucosespiegel angepasst ist.

21. Dosiervorrichtung nach Anspruch 20, **dadurch gekennzeichnet, dass** die Schaltung zum Steuern der Erzeugung von Steuersignalen zu einer Pumpe in der Dosiervorrichtung angepasst ist.

## Revendications

1. Un doseur (1) comprenant une seringue (2) avec une aiguille (3) qui s'étend au-delà du doseur, ledit doseur comprenant, au voisinage de l'aiguille, une face de contact (40) pour venir en contact avec une surface ou un tissu (8) dans lequel doit être insérée l'aiguille, et des moyens détecteurs, **caractérisé en ce qu'**il est prévu sur ladite face de contact des moyens détecteurs capables de détecter un rythme cardiaque.

2. Un doseur selon la revendication 1, **caractérisé en ce que** le doseur comprend des moyens à électrodes additionnels capables de détecter un rythme cardiaque.

3. Un doseur selon la revendication 2, **caractérisé en ce que** les moyens à électrodes additionnels sont prévus sur une poignée (6) du doseur.

4. Un doseur selon la revendication 1 à 3, **caractérisé en ce que** l'aiguille comprend des moyens à électrodes additionnels.

5. Un doseur selon les revendications 1 à 4, **caractérisé en ce que** les moyens à électrodes sont sensibles à des signaux électriques.

6. Un doseur selon les revendications 1 à 4, **caractérisé en ce que** les moyens détecteurs sont sensibles à des signaux optiques.

7. Un doseur selon la revendication 5, **caractérisé en ce que** le doseur comprend des circuits électriques pour générer et pour recevoir des signaux électriques pour mesurer l'impédance entre des électrodes desdits moyens à électrodes.

8. Un doseur selon la revendication 6, **caractérisé en ce que** le doseur comprend un émetteur pour générer et émettre de la lumière et des moyens détecteurs pour détecter de la lumière.

9. Un doseur selon les revendications 7 à 8, **caractérisé en ce que** les signaux sont modulés.

10. Un doseur selon les revendications 7 à 8, **caractérisé en ce que** le doseur contient un circuit de calcul et un circuit de stockage aptes à traiter et à mémoriser la valeur desdits signaux.

11. Un doseur selon les revendications 1 à 10, **caractérisé en ce que** le doseur possède des moyens pour recevoir des signaux externes, et **en ce que** le circuit dans le doseur est adapté pour combiner les signaux externes avec des signaux provenant desdits moyens détecteurs.

12. Un doseur selon les revendications 1 à 11, **caractérisé en ce que** le doseur contient des moyens pour une communication sans fil.

13. Un doseur selon les revendications 1 à 12, **caractérisé en ce que** les moyens détecteurs sont combinés avec des moyens de programme à réseau neuronal pour le traitement du signal.

14. Un doseur selon la revendication 13, **caractérisé en ce que** le programme à réseau neuronal est exécuté sur un ordinateur externe relié au doseur via une liaison de communication sans fil.

15. Un doseur selon la revendication 14, **caractérisé en ce que** lesdits moyens à électrodes comprennent une paire d'électrodes mutuellement étroitement espacées.

16. Un doseur selon les revendications 9 à 13, **caractérisé en ce que** le doseur est adapté pour estimer des signaux d'électrocardiogramme.

17. Un doseur selon les revendications 9 à 13, **caractérisé en ce que** le circuit est adapté pour reconnaître des formes de signal spécifiques.

18. Un doseur selon les revendications 9 à 13, **caractérisé en ce que** le circuit est adapté pour déterminer des rythmes d'impulsions.

19. Un doseur selon la revendication 5, **caractérisé en ce que** le circuit est adapté pour calculer le niveau de glucose sanguin.

20. Un doseur selon la revendication 19, **caractérisé en ce que** le circuit est adapté pour calculer des doses qui dépendent du niveau de glucose sanguin calculé.

21. Un doseur selon la revendication 20, **caractérisé en ce que** le circuit est adapté pour contrôler la production de signaux de contrôle vers une pompe dans le doseur.
